(19) Europäisches Patentamt
European Patent Office
Office européen des brevets

(11) **EP 4 344 770 A2**

(12) **DEMANDE DE BREVET EUROPEEN**

(43) Date de publication:
**03.04.2024 Bulletin 2024/14**

(21) Numéro de dépôt: **24157456.5**

(22) Date de dépôt: **24.05.2019**

(51) Classification Internationale des Brevets (IPC):
***B01J 13/00*** *(2006.01)*

(52) Classification Coopérative des Brevets (CPC):
**B01J 13/0026; B01J 13/0039; C09K 23/00**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.05.2018 FR 1854366**

(62) Numéro(s) de document de la (des) demande(s) initiale(s) en application de l'article 76 CBE:
**19725378.4 / 3 801 867**

(71) Demandeurs:
• **Centre National de la Recherche Scientifique (CNRS)**
**75016 Paris (FR)**
• **PARIS SCIENCES ET LETTRES**
**75006 Paris (FR)**
• **SORBONNE UNIVERSITE**
**75006 Paris (FR)**

(72) Inventeurs:
• **FATTACCIOLI, Jacques**
**75005 PARIS (FR)**
• **PINON, Léa**
**94300 VINCENNES (FR)**
• **MONTEL, Lorraine**
**94230 CACHAN (FR)**

(74) Mandataire: **Regimbeau**
**20, rue de Chazelles**
**75847 Paris Cedex 17 (FR)**

Remarques:
Cette demande a été déposée le 13-02-2024 comme demande divisionnaire de la demande mentionnée sous le code INID 62.

(54) **PARTICULES COLLOIDALES FONCTIONNALISEES DE MANIERE HOMOGENE PAR DES BIOMOLECULES**

(57) La présente invention a trait à un procédé d'obtention d'un colloïde comprenant des particules colloïdales liquides fonctionnalisées, un tel colloïde, ainsi que ses utilisations.

**EP 4 344 770 A2**

Processed by Luminess, 75001 PARIS (FR)

## Description

La présente invention concerne un procédé d'obtention d'un colloïde comprenant des particules colloïdales liquides fonctionnalisées.

Caractérisées par une interface fluide et déformable, les gouttes (encore appelées particules) colloïdales liquides sont intéressantes à bien des points de vue : il est possible de faire varier leur taille, leur composition en volume et en surface à façon, et notamment de les fonctionnaliser avec des motifs moléculaires qui peuvent être fluorescents, ou reconnus par les récepteurs membranaires des cellules.

Des gouttes d'émulsions fonctionnalisées ont ainsi récemment été utilisées comme surfaces biomimétiques pour la modélisation de l'adhésion cellulaire [1-3] ou comme sondes cellulaires pour étudier la phagocytose [4].

Dans la littérature, les gouttes d'émulsion fonctionnalisées par des lipides sont généralement obtenues par la dissolution directe des lipides dans une huile suivie de la fabrication des gouttes [1, 5-6], en utilisant un solvant de support insoluble dans l'eau tel que le chloroforme pour le mélange des lipides avec l'huile avant de procéder à l'émulsification [4, 7], ou par la dissolution des lipides dans l'eau en présence des gouttes après fragmentation [8].

Peu d'études se sont intéressées à la mesure de l'homogénéité de l'insertion des lipides à la surface des gouttes à l'échelle de la suspension entière, mais celles qui l'ont fait ont mis en évidence une importante variation d'une goutte à l'autre au sein d'une même émulsion dans le cas où les lipides sont dissous dans la phase hydrophobe avant émulsification. Pour les lipides dissous directement dans l'eau, leur faible solubilité et leur tendance à former des agrégats métastables tels que des liposomes constituent également une limitation en termes d'efficacité de fonctionnalisation.

Afin de pallier ces problèmes, les inventeurs ont mis au point un nouveau protocole de fonctionnalisation de gouttes permettant d'améliorer l'homogénéité et la cinétique de l'insertion des lipides à la surface des gouttes au sein d'une émulsion donnée.

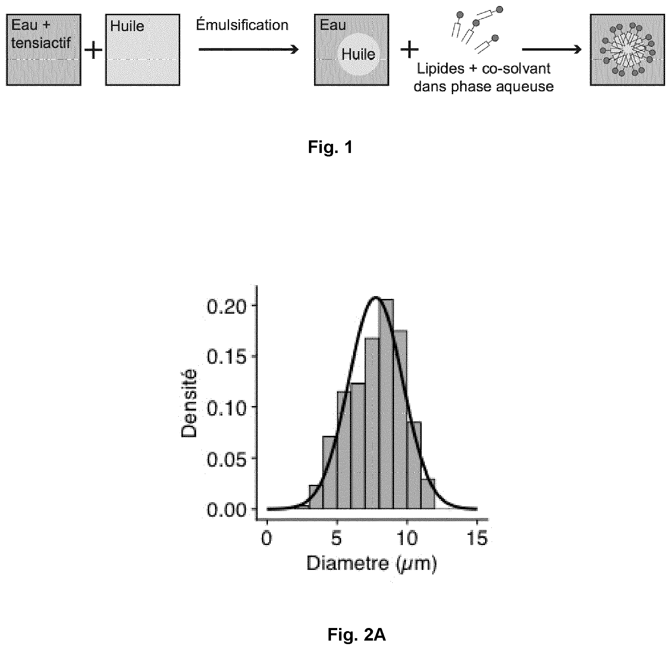

Au lieu de dissoudre les lipides dans l'huile, avec ou sans solvant porteur, avant émulsification, le procédé objet de la présente invention implique la fabrication préalable des gouttes, suivie de l'insertion des lipides à la surface des gouttes dans un second temps (Fig. 1). Il repose sur l'utilisation d'un co-solvant polaire favorisant la dissolution des lipides dans la phase continue aqueuse, et permet d'obtenir des gouttes qui se distinguent par une très grande homogénéité et rapidité de fonctionnalisation.

De telles gouttes peuvent notamment être utilisées pour la calibration de cytomètre en flux.

Dans un cytomètre en flux, des objets individuels tels que des cellules, des billes ou des bactéries sont éclairés par un faisceau laser et plusieurs paramètres sont enregistrés pour chacun d'entre eux : intensités de diffusion aux petits angles (en anglais « Forward Scatter Chanel », FSC) et de diffusion latérale (en anglais « Side Scatter Chanel », SSC), qui donnent des informations sur la taille et la forme des objets, et les intensités de fluorescence. Les paramètres liés à la fluorescence sont utilisés pour quantifier, classer et trier les objets en présence de composés fluorescents intrinsèques ou extrinsèques.

Un cytomètre en flux nécessite un alignement optique rigoureux de ses composants et un calibrage précis des sources lumineuses et des détecteurs pour fournir des mesures fiables. La calibration s'effectue couramment en utilisant des billes micrométriques fluorescentes. Celles-ci sont caractérisées par une forte monodispersité en taille, obtenue grâce à leur mode de synthèse, et une très faible variation de fluorescence au sein d'un même échantillon. Ces particules solides ont néanmoins tendance à s'agréger au cours du temps de stockage, ce qui rend les mesures plus difficiles à réaliser.

Plus récemment, il a été proposé d'utiliser des émulsions calibrées en taille et rendues fluorescentes en dissolvant un fluorophore dans le volume de la phase hydrophobe ou en fonctionnalisant la surface des gouttes avec des molécules ou biomolécules fluorescentes. Le fait que les protocoles traditionnels ne permettent pas d'obtenir une bonne homogénéité de fluorescence constitue cependant un verrou technologique, que la présente invention permet de lever.

La présente invention concerne donc en premier lieu un procédé d'obtention d'un colloïde comprenant des particules colloïdales liquides fonctionnalisées comprenant les étapes suivantes :

a) la dispersion d'une huile 1 dans une solution aqueuse 2 comprenant un tensioactif de fragmentation, aboutissant à l'obtention d'une émulsion 3 comprenant des gouttes d'huile en suspension dans une phase aqueuse ;

b) la dissolution de lipides destinés à fonctionnaliser lesdites gouttes d'huile dans un solvant aprotique polaire, aboutissant à l'obtention d'une solution de fonctionnalisation 4 ;

c) la préparation d'un mélange de fonctionnalisation 5, comprenant l'émulsion 3 et la solution de fonctionnalisation 4, la fraction volumique du solvant aprotique polaire dans ledit mélange de fonctionnalisation 5 étant comprise entre 1 et 15 %, de préférence entre 5 et 15 %, avantageusement entre 8 et 15 % ;

d) l'incubation du mélange de fonctionnalisation 5, au cours de laquelle au moins une partie des lipides initialement présents dans la solution de fonctionnalisation 4 s'adsorbent à la surface des gouttes d'huile initialement présentes dans l'émulsion 3 ; et

e) l'élimination des lipides non adsorbés au cours de l'étape d) ;

permettant ainsi d'obtenir un colloïde comprenant des particules colloïdales liquides fonctionnalisées consistant en les gouttes d'huile obtenues à l'issue de l'étape a) à la surface desquelles sont adsorbés les lipides au cours de l'étape d).

Toute huile connue de l'homme du métier pour former des émulsions huile dans eau peut être utilisée lors de l'étape a). En particulier, l'huile 1 dispersée dans la solution aqueuse 2 peut être une huile minérale, une huile végétale telle que l'huile de soja, une huile siliconée, une huile halogénée (notamment une huile fluorée, une huile iodée ou une huile bromée), une huile animale comme le squalène ou un mélange de celles-ci.

Le coeur liquide des gouttes d'huile peut comprendre un ou plusieurs agents actifs, choisis parmi les agents cosmétiques, pharmaceutiques, comestibles, ou lubrifiants, qui peuvent être hydrophiles ou hydrophobes. Le coeur liquide peut éventuellement comprendre des particules solides en suspension, telles que des nanoparticules métalliques, des particules minérales ou des particules composites par exemple. Avantageusement, lorsqu'elles sont présentes, la taille desdites particules est comprise de 10 nm à 10 $\mu$m. Le coeur liquide peut éventuellement comprendre des particules liquides en suspension, telles que des gouttes d'émulsion de type eau-dans-huile, la taille desdites particules est comprise de 10 nm à 10 $\mu$m.

Dans un mode de réalisation particulier, l'huile 1 correspond à une huile végétale, notamment une huile de soja.

Dans un mode de réalisation particulier, l'huile 1 correspond à une huile minérale comprenant optionnellement des nanoparticules de $Fe_2O_3$ en suspension.

Par « tensioactif de fragmentation » on entend, au sens de la présente invention, toute molécule connue de l'homme du métier facilitant la dispersion de l'huile 1 dans la solution aqueuse 2. Il peut s'agir d'un tensioactif anionique, cationique, zwitterionique ou non-ionique, d'origine naturelle ou synthétique.

Parmi les tensioactifs anioniques, on peut notamment citer les sels (e.g. sodium) de sulfate d'alcool gras linéaire ou ramifié, saturé ou insaturé, de 8 à 20 atomes de carbone, tel que le laurylsulfate de sodium (également appelé dodécylsulfate de sodium ou SDS).

Parmi les tensioactifs cationiques, on peut notamment citer le bromure ou chlorure de cétrimonium (également appelés bromure et chlorure d'hexadécyltriméthylammonium ou CTAB et CTAC).

Parmi les tensioactifs zwitterioniques, on peut notamment citer les bétaïnes telles que la coco-bétaïne (également appelé bétaïne de cocamidopropyle ou CAPB).

Parmi les tensioactifs non-ioniques, on peut notamment citer les esters de sorbitane polyoxyéthyléniques (connus sous le nom commercial « Tween »), les éthers de polyéthylèneglycol (en particulier les séries commerciales « Brij » et « Triton »), les poloxamères (connus sous le nom commercial « Pluronic »), tel que le Pluronic F68.

Dans un mode de réalisation particulier, le tensioactif de fragmentation est un poloxamère tel que le Pluronic F68.

L'étape a) est une étape d'émulsification, qui peut être mise en oeuvre selon toutes les méthodes bien connues de l'homme du métier. La dispersion de l'huile 1 dans la solution aqueuse 2 peut notamment être réalisée par émulsification manuelle, à l'aide de membranes, d'un émulsificateur de Couette ou d'un dispositif microfluidique, ou selon la méthode décrite par Obey et Vincent [J. Colloid Interface Sci. 163, 454 (1994)].

A l'issue de l'étape a), les gouttes d'huile obtenues en suspension dans la phase aqueuse de l'émulsion 3 ont un diamètre compris entre 1 et 20 $\mu$m, en particulier compris entre 2 et 15 $\mu$m, avantageusement entre 3 et 10 $\mu$m.

Le diamètre des gouttes est déterminé par microscopie et analyse d'image, comme décrit dans les Exemples.

Par « solvant aprotique polaire », on entend, au sens de la présente invention un solvant qui possède un moment dipolaire non nul et qui ne comporte aucun atome d'hydrogène acide, c'est-à-dire aucun atome d'hydrogène lié à un hétéroatome tel que N, O ou S. Il peut notamment s'agir d'une cétone, d'un sulfoxyde, d'un amide N,N-disubstitué, d'un ester, d'une amine tertiaire, ou d'un hétérocycle.

Par « cétone », on entend au sens de la présente invention un composé $R_1$-CO-$R_2$, dans lequel $R_1$ et $R_2$ sont des groupements $(C_1$-$C_6)$alkyle, identiques ou différents. A titre d'exemple, on peut citer la propanone et la butanone.

Par « sulfoxyde », on entend au sens de la présente invention un composé $R_3$-SO-$R_4$, dans lequel $R_3$ et $R_4$ sont des groupements $(C_1$-$C_6)$alkyle, identiques ou différents. A titre d'exemple, on peut citer le diméthylsulfoxyde (DMSO).

Par « amide N,N-disubstitué », on entend au sens de la présente invention un composé $R_5$-CO- $NR_6R_7$, dans lequel $R_5$ est un atome d'hydrogène ou un groupement $(C_1$-$C_6)$alkyle, et $R_6$ et $R_7$ sont des groupements $(C_1$-$C_6)$alkyle, identiques ou différents. A titre d'exemple, on peut citer le N,N-diméthylformamide (DMF).

Par « ester », on entend au sens de la présente invention un composé $R_9$-COO-$R_{10}$, dans lequel $R_9$ et $R_{10}$ sont des groupements $(C_1$-$C_6)$alkyle, identiques ou différents. L'ester peut en particulier être un acétate, c'est-à-dire un composé $CH_3COO$-$R_{10}$. A titre d'exemple, on peut citer l'acétate d'éthyle.

Par « amine tertiaire », on entend au sens de la présente invention un composé $NR_{11}R_{12}R_{13}$, dans lequel $R_{11}$, $R_{12}$ et $R_{13}$ sont des groupements $(C_1$-$C_6)$alkyle, identiques ou différents. A titre d'exemple, on peut citer la triéthylamine.

Par groupement « $(C_1$-$C_6)$alkyle », on entend, au sens de la présente invention, une chaîne hydrocarbonée saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par « hétérocycle » on entend, au sens de la présente invention, un cycle saturé ou non, aromatique ou non, et contenant un ou plusieurs hétéroatomes, tels que par exemple des atomes de soufre, azote ou oxygène. Il peut notamment s'agir d'un hétérocycle à 5 ou 6 chaînons contenant avantageusement 1 à 4, encore plus avantageusement 1 ou 2, hétéroatomes. A titre d'exemple, on peut citer la pyridine.

En particulier, le solvant aprotique polaire est sélectionné dans le groupe constitué du DMSO, de l'acétate d'éthyle, de l'acétonitrile, de la pyridine, de la butanone, de la triéthylamine, du N,N-diméthylformamide (DMF), et de leurs mélanges. De préférence, il s'agit d'un sulfoxyde tel que le DMSO.

Tout lipide amphiphile connu de l'homme du métier, naturel ou synthétique, peut être utilisé lors de l'étape b) aboutissant à l'obtention de la solution de fonctionnalisation 4.

Il peut s'agir d'un lipide fluorescent, ou non. Parmi les lipides fluorescents, on peut notamment citer la 1,2-dioleoyl-sn-glycero-3-phosphoethanolamine carboxyfluorescéine (également appelée DOPE CF).

Dans un mode de réalisation, les lipides sont des phospholipides, en particulier des phosphoglycérides, des sphingolipides phosphorylés ou des phosphatidylinositols.

Parmi les phosphoglycérides, on peut notamment citer les phosphatidyléthanolamines, telles que la 1,2-dioléoyl-sn-glycéro-3-phosphoéthanolamine (DOPE) et la 1,2-distéaroyl-sn-glycéro-3-phosphoéthanolamine (DSPE); les phosphatidylcholines et les phosphatidylsérines.

Parmi les sphingolipides phosphorylés, on peut notamment citer la sphingosine-1-phosphate.

Parmi les phosphatidylinositols, on peut notamment citer les glycophosphatidylinositols (GPI).

Avantageusement, les lipides sont des phospholipides, en particulier des phosphatidyléthanolamines telles que DOPE ou DSPE.

Dans un mode de réalisation particulier, les lipides comportent au moins un groupement fluorophore et/ou au moins une biomolécule, pouvant être greffé(e) au moyen d'un linker.

Ledit linker peut être initialement présent dans le lipide utilisé lors de l'étape b), ou greffé ultérieurement.

Il peut comprendre par exemple une chaîne polyéthylène glycol (PEG), ou un groupement nitriloacétate (NTA) optionnellement complexé à du nickel (Ni-NTA). Avantageusement, il s'agit d'une chaîne PEG.

Comme groupement fluorophore, on peut citer à titre d'exemple la 6-carboxyfluorescéine (CF), la fluorescéine, la rhodamine, les fluorophores des gammes commerciales « Alexa Fluor » et « DyLight », avantageusement la 6-carboxyfluorescéine.

La biomolécule peut être notamment une vitamine, telle que la biotine, un peptide, tel que l'acide arginyl glycyl aspartique (RGD), une protéine, telle que la streptavidine, une enzyme, un anticorps, notamment une immunoglobuline de type G (igG) ou une association de celles-ci.

Elle peut être fluorescente ou non, et sa fluorescence peut être intrinsèque ou résulter de la présence d'un groupement fluorophore.

Elle peut être initialement comprise dans le lipide utilisé lors de l'étape b), ou greffée ultérieurement comme décrit ci-après.

Avantageusement, la biomolécule est une biotine. Dans la suite de la description, un lipide greffé d'une biotine au moyen d'une chaîne PEG pourra être désigné par le terme « lipide biotinylé ».

Dans un autre mode de réalisation particulier, les lipides sont des phospholipides, en particulier des phosphatidyléthanolamines telles que DOPE ou DSPE, éventuellement greffés d'au moins un groupement fluorophore et/ou d'au moins une biomolécule, optionnellement au moyen d'un linker.

En particulier, les lipides sont sélectionnés dans le groupe constitué de DOPE-CF, DSPE-PEG-RGD, DSPE-PEG-Biotine et leurs mélanges. Avantageusement, les lipides sont sélectionnés dans le groupe constitué de DOPE-CF, DSPE-PEG-Biotine et leurs mélanges.

Dans un mode de réalisation, un tensioactif de dispersion est ajouté à l'étape c).

Par « tensioactif de dispersion » on entend, au sens de la présente invention, toute molécule amphiphile permettant d'éviter la formation d'agrégats.

De préférence, le tensioactif de dispersion optionnellement ajouté à l'étape c) est non-dénaturant vis-à-vis des protéines.

En particulier, il s'agit d'un tensioactif non-ionique, notamment un ester de sorbitane polyoxyéthylénique ou un éther de polyéthylèneglycol (série « Tween » ou « Brij »), ou d'un poloxamère, ou d'un tensioactif zwitterionique, telle qu'une sulfobétaïne.

Avantageusement, le tensioactif de dispersion ajouté à l'étape c) est le Tween 20 ou le Pluronic F68.

Lorsqu'un tensioactif de dispersion est ajouté à l'étape c), sa concentration dans le mélange de fonctionnalisation 5 est inférieure à 5 fois sa concentration micellaire critique (CMC), de préférence inférieure à 2 fois sa CMC, notamment inférieure à 1,5 fois sa CMC, avantageusement inférieure ou égale à sa CMC.

Dans un mode de réalisation, une solution tampon 6 est ajoutée à l'étape c). De préférence, son pH est compris entre 5 et 9, notamment entre 6 et 8, par exemple son pH vaut environ 7. Il peut notamment s'agir d'un tampon phosphate.

La fraction volumique du solvant aprotique polaire dans le mélange de fonctionnalisation 5 obtenu lors de l'étape c) est comprise entre 1 et 15 %, de préférence entre 5 et 15 %, avantageusement entre 8 et 15 %.

Dans un mode de réalisation, l'étape d) d'incubation est réalisée à une température comprise entre 15 °C et 40 °C, de préférence entre 20 °C et 30 °C, et avantageusement pendant une durée comprise entre 10 min et 10h, de préférence entre 30 min et 5h, avantageusement entre 1h et 3h.

Dans un mode de réalisation particulier, l'huile 1 dispersée dans la solution aqueuse 2 comprend des nano-particules de $Fe_2O_3$ en suspension. La mise en oeuvre d'un tel procédé selon l'invention permet ainsi d'obtenir un colloïde comprenant des particules colloïdales liquides fonctionnalisées magnétiques.

Dans ce mode de réalisation, l'huile 1 est de préférence une huile minérale.

Dans un mode de réalisation particulier, l'huile 1 dispersée dans la solution aqueuse 2 a une densité supérieure à 1. La mise en oeuvre d'un tel procédé selon l'invention permet ainsi d'obtenir un colloïde comprenant des particules colloïdales liquides fonctionnalisées plus denses que la phase aqueuse du colloïde.

Dans ce mode de réalisation, l'huile 1 est de préférence une huile halogénée.

Dans un mode de réalisation, le procédé selon l'invention comprend après l'étape e) une étape supplémentaire f) de greffage de biomolécules sur les lipides adsorbés à la surface des gouttes d'huile.

Les biomolécules greffées lors de cette étape f) peuvent être notamment des vitamines, des peptides, des protéines, telle que la streptavidine, des enzymes, des anticorps, notamment des immunoglobulines de type G (igGs).

Elles peuvent être fluorescentes ou non, et leur fluorescence peut être intrinsèque ou résulter de la présence d'un groupement fluorophore.

En particulier, lorsque les lipides adsorbés à la surface des gouttes d'huile sont des lipides biotinylés, des molécules de streptavidine, avidine, avidine monomérique, neutravidine ou des igGs anti-biotine peuvent être greffées au cours de l'étape f).

Un second objet de la présente invention concerne un colloïde comprenant des particules colloïdales liquides fonctionnalisées susceptible d'être obtenu par le procédé précédemment décrit.

Par ailleurs, la présente invention a également pour objet un colloïde comprenant des particules colloïdales liquides fonctionnalisées en suspension dans un liquide,

- ledit liquide étant constitué d'une solution aqueuse comprenant un solvant aprotique polaire, la fraction volumique dudit solvant aprotique polaire dans ledit liquide étant comprise entre 1 et 15 %, de préférence entre 5 et 15 %, avantageusement entre 8 et 15 % ; et
- lesdites particules colloïdales liquides fonctionnalisées consistant en des gouttes d'huile à la surface desquelles sont adsorbés des lipides.

En particulier, le solvant aprotique polaire est tel que défini précédemment et avantageusement sélectionné dans le groupe constitué du DMSO, de l'acétate d'éthyle, de l'acétonitrile, de la pyridine, de la butanone, de la triéthylamine, du N,N-diméthylformamide (DMF), et de leurs mélanges. De préférence, il s'agit d'un sulfoxyde tel que le DMSO.

L'huile peut être une huile minérale, une huile végétale telle que l'huile de soja, une huile siliconée, une huile halogénée (notamment une huile fluorée, une huile iodée ou une huile bromée), ou un mélange de celles-ci.

Dans un mode de réalisation particulier, les gouttes d'huile sont des gouttes d'huile minérale et comprennent des nanoparticules de $Fe_2O_3$ en suspension.

Dans un autre mode de réalisation particulier, l'huile a une densité supérieure à 1. De préférence, il s'agit d'une huile halogénée.

Tout lipide amphiphile connu de l'homme du métier, naturel ou synthétique, peut être adsorbé à la surface des gouttes d'huile.

Il peut s'agir d'un lipide fluorescent, ou non. Parmi les lipides fluorescents, on peut notamment citer la 1,2-dioleoyl-sn-glycero-3-phosphoethanolamine carboxyfluorescéine (également appelée DOPE CF).

Dans un mode de réalisation, les lipides sont des phospholipides, en particulier des phosphoglycérides, des sphingolipides phosphorylés ou des phosphatidylinositols.

Parmi les phosphoglycérides, on peut notamment citer les phosphatidyléthanolamines, telles que la 1,2-dioléoyl-sn-glycéro-3-phosphoéthanolamine (DOPE) et la 1,2-distéaroyl-sn-glycéro-3-phosphoéthanolamine (DSPE); les phosphatidylcholines et les phosphatidylsérines.

Parmi les sphingolipides phosphorylés, on peut notamment citer la sphingosine-1-phosphate.

Parmi les phosphatidylinositols, on peut notamment citer les glycophosphatidylinositols (GPI).

Avantageusement, les lipides sont des phospholipides, en particulier des phosphatidyléthanolamines telles que DOPE ou DSPE.

Dans un mode de réalisation particulier, les lipides comportent au moins un groupement fluorophore et/ou au moins une biomolécule, pouvant être greffé(e) au moyen d'un linker.

Ledit linker peut comprendre une chaîne polyéthylène glycol (PEG) ou un groupement nitriloacétate (NTA) optionnellement complexé à du nickel (Ni-NTA). Avantageusement, il s'agit d'une chaîne PEG.

Comme groupement fluorophore, on peut citer à titre d'exemple la 6-carboxyfluorescéine (CF), la fluorescéine, la rhodamine, les fluorophores des gammes commerciales « Alexa Fluor » et « DyLight », avantageusement la 6-carboxyfluorescéine.

La biomolécule peut être notamment une vitamine, telle que la biotine, un peptide, tel que l'acide arginyl glycyl aspartique (RGD), une protéine, telle que la streptavidine, une enzyme, un anticorps, notamment une immunoglobuline de type G (igG) ou une association de celles-ci.

Elle peut être fluorescente ou non, et sa fluorescence peut être intrinsèque ou résulter de la présence d'un groupement fluorophore.

Avantageusement, la biomolécule est une biotine, typiquement greffée au moyen d'une chaîne PEG.

Le fragment PEG-biotine peut alors représenter lui-même un linker, sur lequel peut être greffée une biomolécule présentant une affinité pour biotine, telle que la streptavidine, l'avidine, l'avidine monomérique, la neutravidine ou une igG anti-biotine.

Dans un autre mode de réalisation particulier, les lipides sont des phospholipides, en particulier des phosphatidyléthanolamines telles que DOPE ou DSPE, éventuellement greffés d'au moins un groupement fluorophore et/ou d'au moins une biomolécule, optionnellement au moyen d'un linker.

En particulier, les lipides sont sélectionnés dans le groupe constitué de DOPE-CF, DSPE-PEG-RGD, DSPE-PEG-Biotine et leurs mélanges. Avantageusement, les lipides sont sélectionnés dans le groupe constitué de DOPE-CF, DSPE-PEG-Biotine et leurs mélanges.

Dans un mode de réalisation particulier, le colloïde est tel que, au sein d'une sous-population de particules ayant un diamètre égal à d +/- 15 %, le coefficient de variation de la quantité de lipide adsorbés à la surface des gouttes est inférieur ou égal à 20 %.

La présente invention concerne de surcroît l'utilisation d'un colloïde tel que décrit précédemment pour le calibrage d'un cytomètre en flux.

La présente invention concerne de surcroît l'utilisation d'un colloïde comprenant des particules colloïdales liquides fonctionnalisées susceptible d'être obtenu par le procédé de la présente invention pour le calibrage d'un cytomètre en flux.

La présente invention concerne en outre l'utilisation d'un colloïde tel que décrit précédemment pour l'étude de leur ingestion par des cellules, telle que la phagocytose par des macrophages. Avantageusement, la densité de l'huile constitutive des particules colloïdales liquides fonctionnalisées est alors supérieure à 1. Typiquement, il s'agit d'une huile halogénée.

La présente invention concerne de surcroît l'utilisation d'un colloïde comprenant des particules colloïdales liquides fonctionnalisées susceptible d'être obtenu par le procédé de la présente invention pour l'étude de leur ingestion par des cellules, telle que la phagocytose par des macrophages. Avantageusement, la densité de l'huile constitutive des particules colloïdales liquides fonctionnalisées est alors supérieure à 1. Typiquement, il s'agit d'une huile halogénée.

La présente invention a également pour objet les réalisations énumérées aux points suivants.

Réalisation 1 : Procédé d'obtention d'un colloïde comprenant des particules colloïdales liquides fonctionnalisées comprenant les étapes suivantes :

a) la dispersion d'une huile 1 dans une solution aqueuse 2 comprenant un tensioactif de fragmentation, aboutissant à l'obtention d'une émulsion 3 comprenant des gouttes d'huile en suspension dans une phase aqueuse ;
b) la dissolution de lipides destinés à fonctionnaliser lesdites gouttes d'huile dans un solvant aprotique polaire, aboutissant à l'obtention d'une solution de fonctionnalisation 4 ;
c) la préparation d'un mélange de fonctionnalisation 5, comprenant l'émulsion 3 et la solution de fonctionnalisation 4, la fraction volumique du solvant aprotique polaire dans ledit mélange de fonctionnalisation 5 étant comprise entre 1 et 15 %, de préférence entre 5 et 15 %, avantageusement entre 8 et 15 % ;
d) l'incubation du mélange de fonctionnalisation 5, au cours de laquelle au moins une partie des lipides initialement présents dans la solution de fonctionnalisation 4 s'adsorbent à la surface des gouttes d'huile initialement présentes dans l'émulsion 3 ; et
e) l'élimination des lipides non adsorbés au cours de l'étape d) ;

permettant ainsi d'obtenir un colloïde comprenant des particules colloïdales liquides fonctionnalisées consistant en les gouttes d'huile obtenues à l'issue de l'étape a) à la surface desquels sont adsorbés les lipides au cours de l'étape d).

Réalisation 2 : Procédé selon la réalisation 1, caractérisé en ce que le solvant aprotique polaire est sélectionné dans le groupe constitué du DMSO, de l'acétate d'éthyle, de l'acétonitrile, de la pyridine, de la butanone, de la triéthylamine, du DMF, et de leurs mélanges, de préférence il s'agit du DMSO.

Réalisation 3 : Procédé selon la réalisation 1 ou 2, caractérisé en ce que l'huile 1 dispersée dans la solution aqueuse 2 au cours de l'étape a) est une huile minérale, une huile végétale telle que l'huile de soja, une huile siliconée, une huile halogénée, ou un mélange de celles-ci.

Réalisation 4 : Procédé selon l'une quelconque des réalisation 1 à 3, caractérisé en ce que les lipides comportent au moins un groupement fluorophore et/ou au moins une biomolécule, telle qu'une biotine, optionnellement greffé(e) au moyen d'un linker tel qu'une chaîne PEG.

Réalisation 5 : Procédé selon l'une quelconque des réalisations 1 à 4, caractérisé en ce que les lipides sont des phospholipides, en particulier des phosphatidyléthanolamines telles que DOPE ou DSPE, éventuellement greffés d'au moins un groupement fluorophore et/ou d'au moins une biomolécule, optionnellement au moyen d'un linker, notamment sélectionnés dans le groupe constitué de DOPE-CF, DSPE-PEG-Biotine et leurs mélanges.

Réalisation 6 : Procédé selon l'une quelconque des réalisations 1 à 5, caractérisé en ce que à l'étape c), un tensioactif de dispersion est ajouté.

Réalisation 7 : Procédé selon l'une quelconque des réalisations 1 à 6, caractérisé en ce que à l'étape c), une solution tampon 6 ayant un pH compris entre 5 et 9 est ajoutée.

Réalisation 8 : Procédé selon l'une quelconque des réalisations 1 à 7, caractérisé en ce que l'étape d) d'incubation est réalisée à une température comprise entre 15 °C et 40 °C, de préférence entre 20 °C et 30 °C, et a une durée comprise entre 10 min et 10h, de préférence entre 30 min et 5h, avantageusement entre 1h et 3h.

Réalisation 9 : Procédé selon l'une quelconque des réalisations 1 à 8, caractérisé en ce que l'huile 1 dispersée dans la solution aqueuse 2 comprend des nanoparticules de $Fe_2O_3$ en suspension, permettant ainsi d'obtenir colloïde comprenant des particules colloïdales liquides fonctionnalisées magnétiques.

Réalisation 10 : Procédé selon l'une quelconque des réalisations 1 à 9, caractérisé en ce qu'il comprend après l'étape e) une étape supplémentaire f) de greffage de biomolécules sur les lipides.

Réalisation 11 : Colloïde comprenant des particules colloïdales liquides fonctionnalisées susceptible d'être obtenu par le procédé selon l'une quelconque des réalisations 1 à 10.

Réalisation 12 : Colloïde comprenant des particules colloïdales liquides fonctionnalisées en suspension dans un liquide, caractérisé en ce que :

- le liquide est constitué d'une solution aqueuse comprenant un solvant aprotique polaire, la fraction volumique dudit solvant aprotique polaire dans ledit liquide étant comprise entre 1 et 15 %, de préférence entre 5 et 15 %, avantageusement entre 8 et 15 % ; et
- les particules colloïdales liquides fonctionnalisées consiste en des gouttes d'huile à la surface desquelles sont adsorbés des lipides.

Réalisation 13 : Colloïde comprenant des particules colloïdales liquides fonctionnalisées selon la réalisation 12, caractérisé en ce que la variation de la quantité de lipides adsorbées à la surface d'une sous-population de particules de diamètre caractérisé par un coefficient de variation inférieur ou égal à 15 % est inférieure ou égale à 20 %.

Réalisation 14 : Utilisation d'un colloïde comprenant des particules colloïdales liquides fonctionnalisées selon l'une quelconque des réalisations 11 à 13 pour le calibrage d'un cytomètre en flux.

Réalisation 15 : Utilisation d'un colloïde comprenant des particules colloïdales liquides fonctionnalisées selon l'une quelconque des réalisations 11 à 13 pour l'étude de leur ingestion par des cellules, telle que la phagocytose par des macrophages, caractérisée en ce que la densité de l'huile constitutive des particules colloïdales liquides fonctionnalisées est supérieure à 1.

**FIGURES**

- La Figure 1 est une représentation schématique du protocole d'obtention de gouttes d'émulsion fonctionnalisées par des lipides selon l'invention.

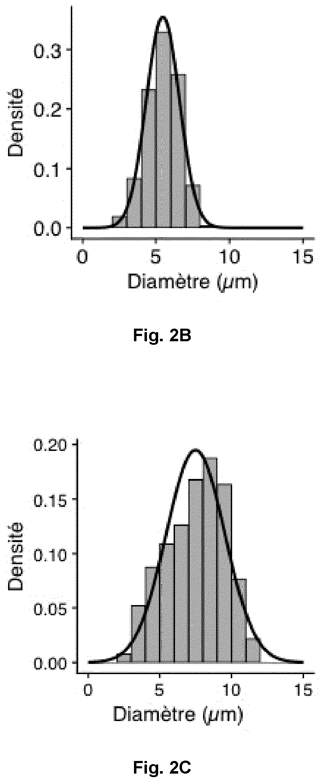

- Les Figures 2A et 2B représentent les distributions de tailles des gouttes au sein des émulsions A (Fig. 2A) et B (Fig. 2B). Les échantillons ont un diamètre égal respectivement à 8 ± 20 % μm et à 5,3 ± 20 % μm.
- La Figure 2C correspond à la distribution de taille d'un sous-échantillon de diamètre 8 ± 1 μm (émulsion C).

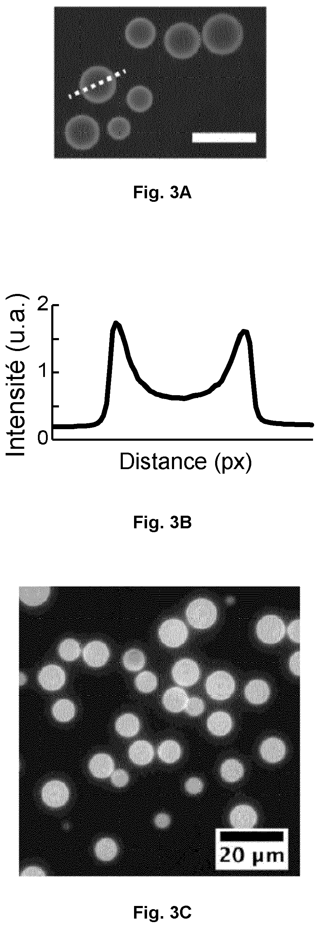

- La Figure 3A correspond à l'image des gouttes fonctionnalisées par DOPE-CF enregistrée par microscopie confocale à disque tournant (barre d'échelle = 15 μm).
- La Figure 3B représente le profil de fluorescence d'une goutte de la figure 3A (voir trait pointillé, Fig. 3A).
- La Figure 3C correspond à l'image de l'émulsion Afonctionnalisée par DOPE-CF prise par microscopie à épifluorescence à champ large avec un objectif 40x. Les gouttelettes montrent une fluorescence homogène du fait de la profondeur de champ de l'objectif (barre d'échelle = 20 μm).

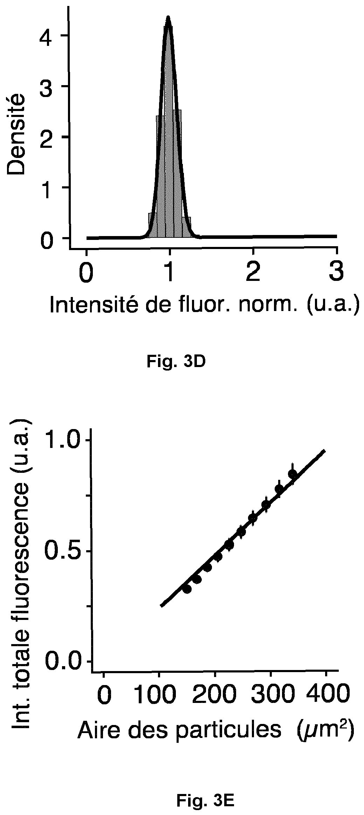

- La Figure 3D représente la distribution de fluorescence d'un sous-échantillon d'une émulsion de diamètre égal à 8 ± 1 μm (émulsion C) fonctionnalisée par DOPE-CF. Les données expérimentales sont mesurées par épifluorescence à champ large et suivent une distribution normale (CV = 8 %). Les intensités de fluorescence sont normalisées par rapport à la valeur moyenne du sous-ensemble.
- La Figure 3E représente l'intensité de fluorescence intégrée des gouttes d'émulsion fonctionnalisées par DOPE-CF (5 éq.) en fonction du carré du rayon des gouttes ($R^2$) et mesurée par microscopie à épifluorescence. Les données expérimentales suivent une fonction linéaire.

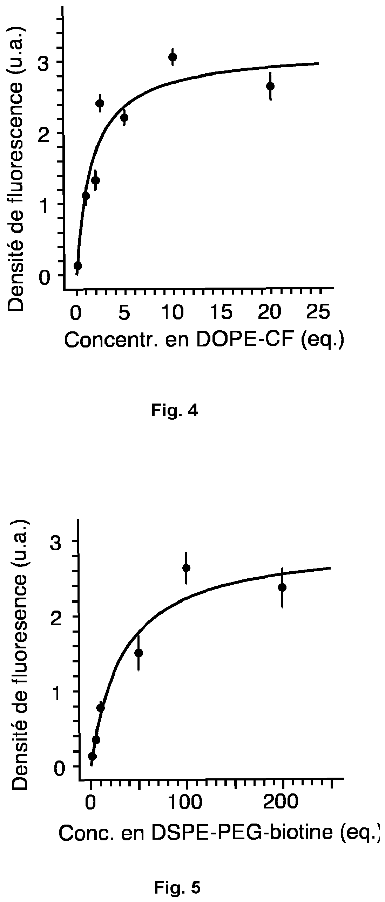

- La Figure 4 correspond à la courbe de titration des gouttes fonctionnalisées par DOPE-CF. La densité de fluorescence des gouttes, c'est-à-dire la fluorescence totale par objet divisée par sa surface sur les images, est représentée en fonction de la concentration en DOPE-CF, exprimée en équivalent de surface par rapport à une monocouche.
- La Figure 5 correspond à la courbe de titration des gouttes fonctionnalisées par DSPE-PEG-biotine par rapport à la concentration de lipides biotinylés. Après insertion du lipide biotinylé non fluorescent, des igGs fluorescentes sont ajoutées à la phase aqueuse à une concentration de saturation de 5 éq.

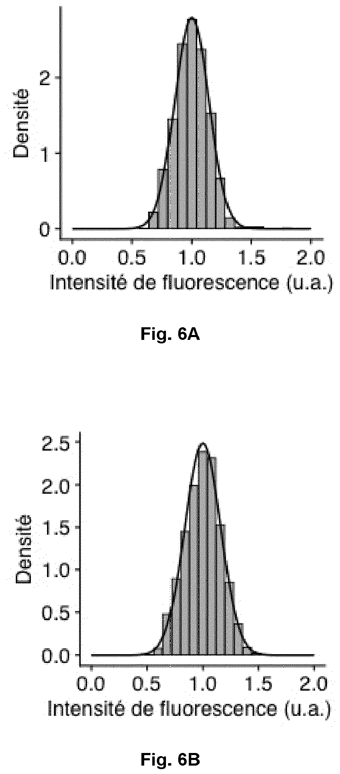

- Les Figures 6A et 6B représentent les distributions de fluorescence obtenues pour les gouttes de l'émulsion C fonctionnalisées par DSPE-PEG-biotine-streptavidine (Fig. 6A) ou DSPE-PEG-biotine-igG (Fig. 6B). Les coefficients de variation sont de 14 % et 16 % respectivement.

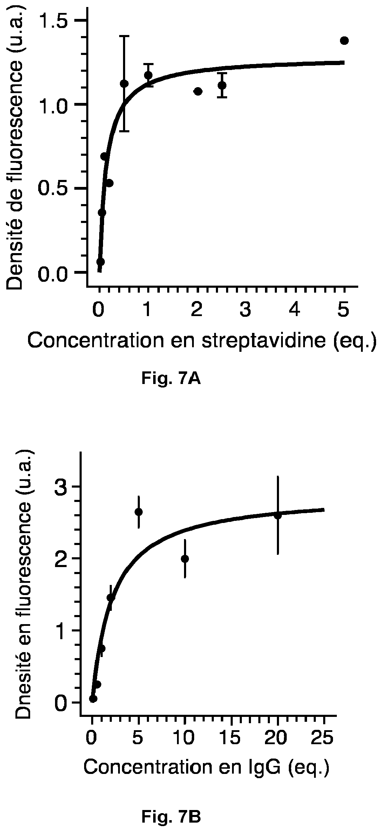

- Les Figures 7A et 7B correspondent aux courbes de titration obtenues pour le greffage de la streptavidine (Fig. 7A) et de l'igG anti-biotine (Fig. 7B) aux gouttes fonctionnalisées par DSPE-PEG-biotine.

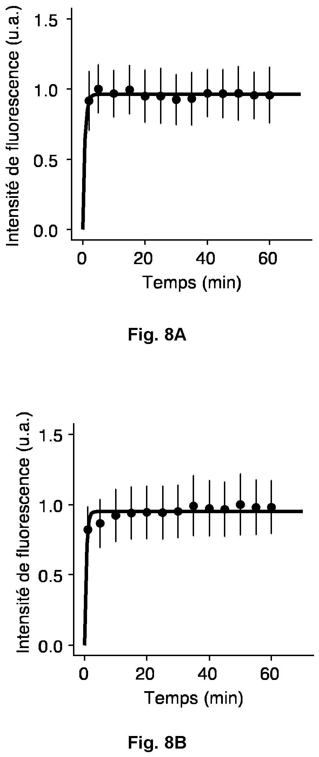

- Les Figures 8A et 8B représentent les cinétiques de greffage de la streptavidine (Fig. 8A) et de l'IgG anti-biotine (Fig. 8B) aux gouttes biotinylées, déterminées par des mesures de fluorescence effectuées avec un cytomètre en flux. Les gouttes ont été biotinylées avec une solution de DSPE-PEG-biotine (100 éq.) contenant 10 % v/v de DMSO. Les barres d'erreur correspondent à l'écart-type calculé à partir de N = 3 expériences indépendantes. Les données expérimentales correspondent à des cinétiques de premier ordre dans les deux cas.

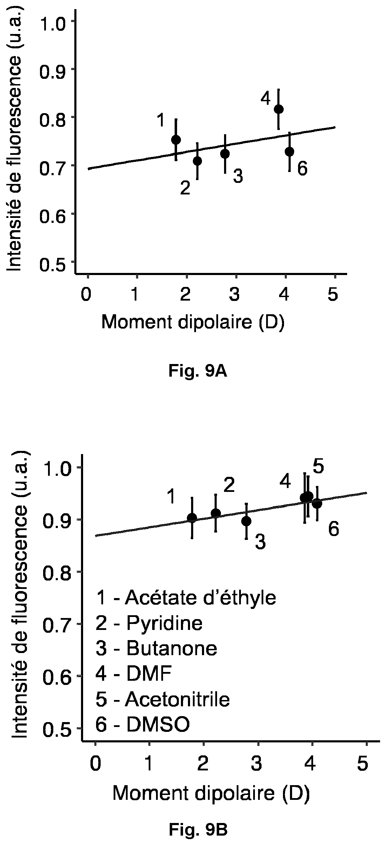

- Les Figures 9A et 9B représentent l'intensité totale de fluorescence mesurée par cytométrie en flux pour des gouttes fonctionnalisées par DOPE-CF (Fig. 9A) ou DSPE-PEG-biotine (Fig. 9B) sur lesquels sont ensuite greffés des igGs fluorescentes, en fonction du moment dipolaire du solvant utilisé dans la solution de fonctionnalisation.

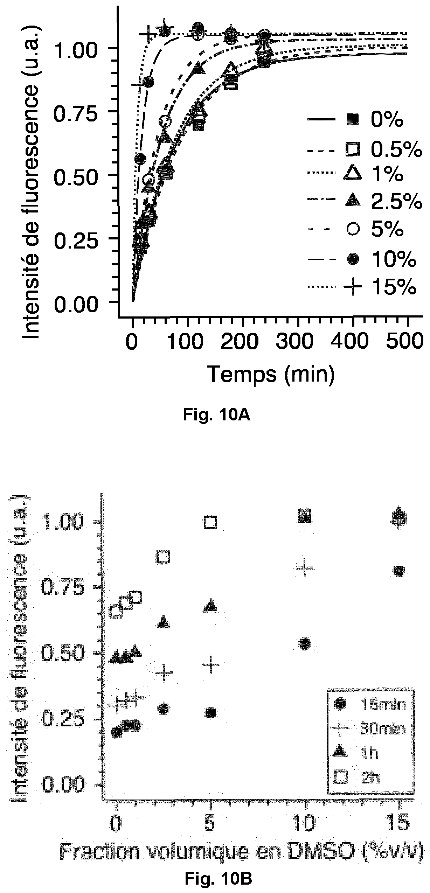

- La Figure 10A représente l'intensité totale de fluorescence mesuré pour des gouttes fonctionnalisées par DOPE-CF, en fonction du temps, pour des fractions volumiques en DMSO dans la phase continue pendant l'étape d'adsorption des lipides variant entre 0 et 15 % v/v. Pour chaque point indiqué sur le graphique, les gouttes ont été fonctionnalisées en utilisant une concentration de DOPE-CF de 2,5 équivalents.
- La Figure 10B représente l'intensité totale de fluorescence mesuré pour des gouttes fonctionnalisées par une concentration de DOPE-CF de 2,5 équivalents, en fonction de la fraction volumique en DMSO dans la phase continue pendant l'étape d'adsorption des lipides variant entre 0 et 15 % v/v, pour des temps de réaction de 15, 30, 60 et 120 min. La fluorescence des gouttes a été mesurée par cytométrie en flux.

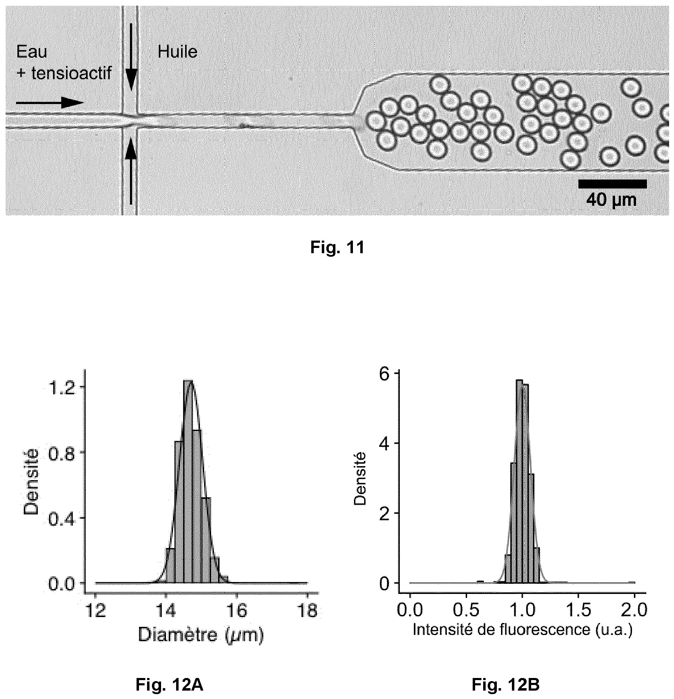

- La Figure 11 représente une image en fond clair du dispositif de focalisation d'écoulement microfluidique utilisé pour la production de gouttelettes magnétiques monodisperses.
- Les Figures 12A et 12B représentent les distributions de tailles (Fig. 12A) et de fluorescence (Fig. 12B) des gouttes magnétiques biotinylées greffées d'igGs anti-biotine fluorescentes obtenus par le dispositif précédent. Les gouttes ont un diamètre égal à 14,7 μm ± 2 % et un CV de fluorescence égal à 10 %.

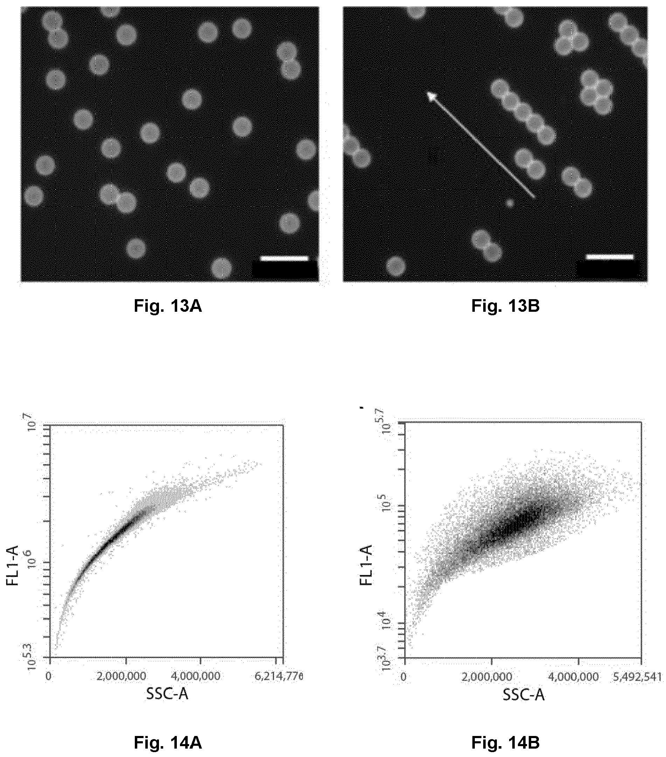

- Les Figures 13A et 13B montrent la répartition de gouttes magnétiques biotinylées conjuguées à des igGs fluorescentes anti-biotine sans (Fig. 13A) ou avec application d'un champ magnétique (Fig. 13B) (barre d'échelle : 30 μm).
- La Figure 14A correspond au diagramme de densité FL vs. SS obtenu par cytométrie en flux dans le cas de gouttes fonctionnalisées par DSPE-PEG-biotine selon le procédé de l'invention puis greffées d'IgGs anti-biotine fluorescentes.
- La Figure 14B correspond au diagramme de densité FL vs. SS obtenu par cytométrie en flux dans le cas de gouttes fonctionnalisées par DSPE-PEG-biotine selon un protocole de l'art antérieur (dans lequel les lipides sont dilués dans l'huile avant émulsification) puis greffées d'igGs anti-biotine fluorescentes.

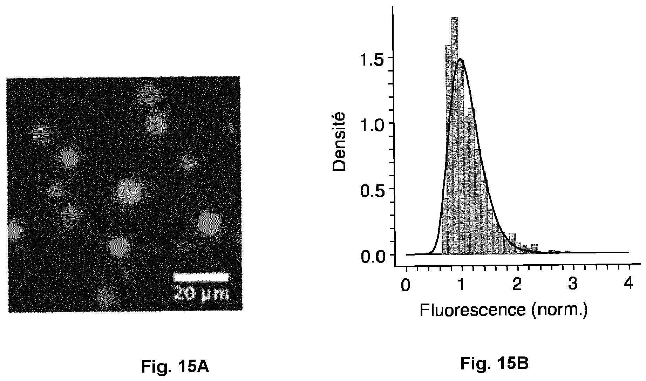

- La Figure 15A correspond à une image prise par microscopie à épifluorescence à champ large de gouttes (diamètre moyen 8 $\mu$m) fonctionnalisées par DSPE-PEG-biotine selon le protocole de l'art antérieur (dans lequel les lipides sont dilués dans l'huile avant émulsification) puis greffées d'igGs anti-biotine fluorescentes (barre d'échelle : 20 $\mu$m).
- La Figure 15B représente la distribution de fluorescence normalisée des gouttes précédentes (Fig. 15A). Les données expérimentales correspondent à une distribution log-normale ($\mu$ = -0,31 ; $\sigma$ = 0,78 ; CV = 92 %). Les intensités de fluorescence sont normalisées par rapport à la valeur moyenne de l'ensemble de la population.

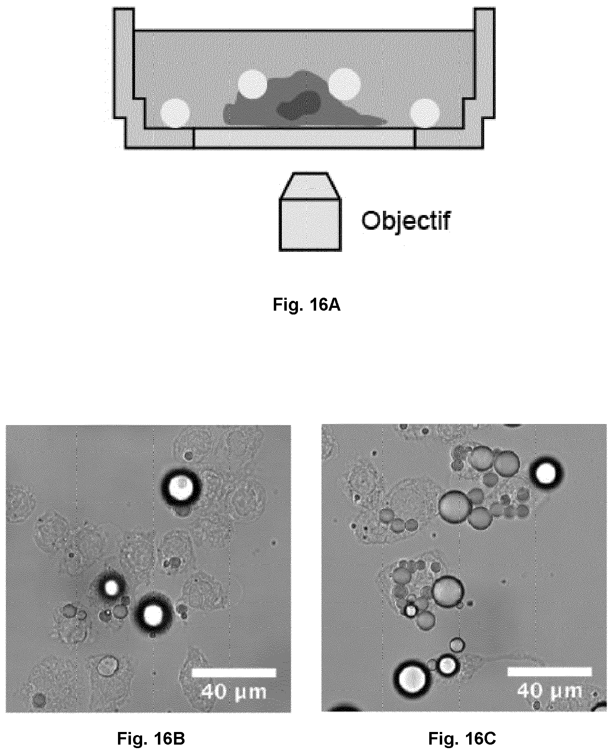

- La Figure 16A correspond à une représentation schématique du dispositif utilisé pendant les expériences de phagocytose. Les cellules sont déposées au fond d'une boite de Pétri munie d'un fond en verre optique. Les gouttes fonctionnalisées sont dispersées dans le milieu de culture, sédimentent grâce à la gravité, entrent en contact avec les cellules et sont éventuellement internalisées.
- La Figure 16B correspond à une image en microscopie de fond clair représentative d'une expérience de phagocytose de gouttes d'huiles plus denses que le milieu de culture fonctionnalisées par des IgG lorsque celles-ci sont mises en contact avec des macrophages de lignée. La figure 16C représente l'expérience contrôle, réalisée avec des gouttes ne présentant pas d'IgG à la surface.

## EXEMPLES

Les abréviations suivantes ont été utilisées :

| | |
|---|---|
| BSA | : albumine sérique bovine |
| CF | : 6-carboxyfluorescéine |
| CMC | : concentration micellaire critique |
| CV | : coefficient de variation |
| DMEM | : milieu Eagle modifié Dulbecco |
| DMF | : N,N-diméthylformamide |
| DMSO | : diméthylsulfoxyde |
| DOPE-CF | : 1,2-dioléoyl-sn-glycéro-3-phosphoéthanolamine-N-(carboxyfluorescéine) |
| DSPE-PEG- biotine | : 1,2-distéaroyl-sn-glycéro-3-phosphoéthanolamine-N-[biotinyl (polyethylene glycol)-2000] |
| IgG | : immunoglobuline G |
| PB | : tampon phosphate |
| PBS | : tampon phosphate salin |
| PDMS | : polydiméthylsiloxane |

### Matériels et Méthodes

A) Matériels

L'huile de soja (CAS No. 8001-22-7), l'huile minérale (CAS No. 8042-47-5), le Pluronic F68 (CAS No. 9003-11-6), le Tween 20 (CAS No. 9005-64-5), l'acide oléique (CAS No. 112-80-1), l'alginate de sodium (CAS No. 9005-38-3), le DMSO, l'acétonitrile, le DMF, la pyridine, la butanone, l'acétate d'éthyle et la triéthylamine ont été achetés auprès de Sigma-Aldrich (St Quentin Fallavier, France).
La DOPE-CF et la DSPE-PEG-biotine ont été achetés auprès d'Avanti Polar Lipids.
L'igG anti-biotine de souris conjuguée à l'Alexa Fluor 488 (Réf. 200-542-211) a été achetée auprès de Jackson Immunoresearch (West Grove, PA, USA).
La streptavidine FluoProbes 488 a été achetée auprès de Interchim.

B) Fabrication de l'émulsion avec un émulsificateur de Couette

De l'huile de soja a été dispersée et émulsifiée à la main dans une phase aqueuse continue contenant 15 % en poids du tensioactif de type copolymère séquencé Pluronic F68 (CMC = 0,03 g.l$^{-1}$) et 1 % en poids d'alginate de sodium à une fraction finale d'huile égale à 75 % en poids. L'émulsion brute a été soumise à un cisaillement dans un appareil à cellule de Couette en suivant la méthode développée par Mason *et al.* [9] pour fabriquer deux échantillons d'émulsions quasi monodisperses avec des diamètres moyens de 8 $\pm$ 2 $\mu$m (émulsion A, Fig. 2A) et 5,5 $\pm$ 2 $\mu$m (émulsion B, Fig. 2B), respectivement. Pour le stockage et la manipulation, les émulsions ont été diluées jusqu'à une fraction d'huile de 60 % en poids avec 1 % en poids de Pluronic F68 dans la phase continue et stockées à 12 °C dans une armoire à refroidissement Peltier pendant plusieurs semaines.

### C) Préparation des solutions de fonctionnalisation

Après évaporation du chloroforme dans lequel les phospholipides sont fournis, les lipides sont d'abord dissous dans un solvant polaire à la concentration de 1 mg/mL pour DOPE-CF et de 10 mg/mL pour DSPE-PEG-Biotine. Des solutions de fonctionnalisation ont été préparées avec du DMSO, de l'acétonitrile, du DMF, de la pyridine, de la butanone, de l'acétate d'éthyle et de la triéthylamine.

### D) Protocole de fonctionnalisation des gouttes avec des phospholipides

Les gouttes en émulsion sont diluées dans un microtube jusqu'à une concentration d'environ $5.10^6$ gouttes.$\mu$L$^{-1}$ dans 200 $\mu$L d'un tampon phosphate (pH = 7,2, 20 mM) complété avec du Tween 20 à sa CMC = 0,006 g.L$^{-1}$ [10]. La suspension est centrifugée et rincée 4 fois avec le tampon phosphate pour diminuer la quantité de Pluronic F68 dans la phase continue. Après la dernière étape de rinçage, la majeure partie de la solution aqueuse est éliminée du microtube pour obtenir 10 $\mu$L d'émulsion. On ajoute ensuite entre 0 et 10% v/v de solution de fonctionnalisation, et pour finir le tampon phosphate avec du Tween 20 (CMC) pour atteindre un volume final de 200 $\mu$L de suspension.

Les gouttes sont incubées pendant 90 minutes à température ambiante en présence des lipides dans la phase continue, et sont finalement rincées plusieurs fois avec le mélange PB/Tween 20 CMC pour éliminer de la solution les phospholipides non adsorbés.

La quantité de phospholipides disponibles initialement dans la phase continue est ajustée en diluant la solution de fonctionnalisation à l'aide de son co-solvant de façon à ce que la fraction volumique du co-solvant soit la même dans toutes les expériences. Dans la plupart, c'est le DMSO qui a été utilisé comme co-solvant, à la fraction volumique de 10 % v/v.

La concentration en lipide dans la phase continue est exprimée en équivalents de surface de gouttes par rapport au nombre de molécules nécessaires pour recouvrir l'interface des gouttes avec une monocouche compacte de lipides. La surface par tête lipidique est de 12 nm$^2$ pour la DSPE-PEG-Biotine [11] et de 0,5 nm$^2$ pour la DOPE-CF [12], ce qui correspond respectivement à un volume de solution de fonctionnalisation égal à 0,04 $\mu$L et 3,55 $\mu$L pour atteindre un équivalent de surface de phospholipides pour une suspension de gouttes de 8 $\mu$m contenant environ $5.10^6$ gouttes.

### E) Greffage d'IgGs anti-biotine sur les gouttes biotinylées

Le couplage des IgGs aux biotines présentes à la surface des gouttes a été obtenu après une incubation de 30 minutes de la suspension de gouttes (200 $\mu$L, $5.10^6$ gouttes) avec une quantité variable d'une solution mère d'igG anti-biotine de souris conjuguée à l'Alexa Fluor 488 (0,8 mg/mL) à température ambiante dans un tampon phosphate (PB, pH = 7,2, 20 mM, Tween 20 à la CMC). Pour des gouttes de 8 $\mu$m, le volume de solution mère à utiliser pour obtenir une concentration correspondant à un équivalent de surface d'igG sur les gouttes est égal à 2,5 $\mu$L si l'on considère une surface moléculaire de 120 nm$^2$ par igG [13]. Les gouttes sont rincées plusieurs fois dans le même tampon pour éliminer les igGs n'ayant pas réagi et observées par microscopie.

### F) Greffage de streptavidine sur des gouttes biotinylées

Les streptavidines sont spécifiquement adsorbées à la surface des gouttes fonctionnalisées par des lipides biotinylés. Le couplage a été obtenu après une incubation de 30 minutes de la suspension de gouttes (200 $\mu$L, $5.10^6$ gouttes) avec une quantité variable d'une solution mère de streptavidine FluoProbes 488 (1 mg/mL) à température ambiante dans un tampon phosphate (PB, pH = 7,2, 20 mM, Tween 20 à la CMC). Pour des gouttes de 8 $\mu$m, le volume de solution mère à utiliser pour obtenir une concentration correspondant à un équivalent de surface de streptavidine sur les gouttes est égal à 5,78 $\mu$L si l'on considère une surface moléculaire de 16,6 nm$^2$ par streptavidine [13].

Les gouttes sont rincées plusieurs fois dans le même tampon pour éliminer la streptavidine n'ayant pas réagi et observées par microscopie.

### G) Microscopie et analyse d'images

Les images en fond clair et fluorescentes ont été acquises sur un microscope Zeiss Axio Observer Z1 (Oberkochen, Allemagne) connecté à une caméra PCO Edge 4.2 sCMOS (PCO, Allemagne). L'épi-illumination a été réalisée avec un système Lumencor Spectra X LED. La distribution des tailles et la fluorescence des gouttes en émulsion ont été mesurées par microscopie et analyse d'image. Tous les calculs ont été effectués avec les logiciels Fidji / ImageJ [5] et R [6]. Les gouttes sont observées avec un objectif Zeiss x40. Les enregistrements de microscopie à disque rotatif ont été réalisés à l'aide d'un microscope Leica SD AF, avec un objectif x40.

H) Cytométrie en flux

Les gouttes d'émulsion ont été caractérisées à l'aide d'un cytomètre BD Accuri C6 (BD Biosciences, New Jersey, USA).

I) Fabrication du dispositif microfluidique

Le dispositif est réalisé en PDMS, en utilisant les techniques standard de lithographie douce [14]. Un master SU-8 (SU-8 20XX, Microchem) a été fabriqué sur une plaquette de silicium à l'aide d'un dispositif de masquage laser à écriture directe (Kloe Dilase 650), puis le moulage PDMS (RTV 615, ratio 1:10 pour l'agent réticulant, RTV 615, Momentive Performance Materials) a été réalisé, suivi d'un traitement thermique à 80 °C pendant deux heures. Les surfaces PDMS et la lamelle de verre (VWR, 50 $\times$ 24 mm) qui ferme le canal ont été traitées avec du plasma $O_2$ (Cute Plasma, Corée) avant de sceller les deux parties de la puce ensemble. Les dimensions du canal à la jonction de focalisation du flux sont: largeur (l) = 10 $\mu$m, hauteur (h) = 12 $\mu$m et longueur (L) = 10 $\mu$m.

J) Synthèse du ferrofluide

Le ferrofluide est constitué de nanoparticules de maghémite ($Fe_2O_3$) produites par co-précipitation dans une phase aqueuse de Fe (II) et de Fe (III) en conditions alcalines, suivant la méthode développée par Massart [15]. La taille des nanoparticules est comprise entre 5 et 20 nm et dépend de la cinétique de la réaction. Pour les expériences décrites ci-après, des particules avec une distribution de taille de 7 $\pm$ 1,6 nm ont été utilisées. Après synthèse, la phase aqueuse est échangée avec du chloroforme en utilisant un excès d'acide oléique jouant le rôle de tensioactif et de stabilisant. La concentration en fer dans le produit final a été caractérisée et est égale à 1,57 M.

**Résultats**

1. Distribution de la fluorescence des gouttes

Un phospholipide fluorescent, la DOPE carboxyfluorescéine (CF), a été dissous dans 10 % v/v de DMSO dans l'eau selon le protocole décrit dans la partie « Matériels et méthodes », section D), pour étudier son insertion à la surface de gouttes d'huile de soja de 8 $\mu$m. La figure 3A représente une image des gouttes en microscopie confocale à disque rotatif. Les gouttes présentent une interface fluorescente homogène et une partie centrale pratiquement noire, ce qui indique que les phospholipides fluorescents ne sont présents qu'à la surface (voir également Fig. 3B). Après la mesure de la distribution de la fluorescence des gouttes au moyen d'un microscope à épifluorescence à fond clair sur plusieurs milliers d'objets, la figure 3D présente la distribution de la fluorescence d'un sous-échantillon d'émulsion (émulsion C, Fig. 2C) ayant un diamètre de 8 $\pm$ 1 $\mu$m. La fluorescence est très homogène et suit une distribution normale avec un écart-type de 9 %.

2. Intensité de fluorescence en fonction de la taille des gouttes.

En utilisant un logiciel d'analyse d'images sur les images d'épifluorescence des gouttes, avec le seuil binaire approprié, il est possible de représenter l'intensité de fluorescence intégrée sur toute la zone planaire des gouttes $I_{Total}$. La figure 3E montre ainsi que $I_{Toal}$ varie linéairement avec le carré du rayon des gouttes. Puisque la fluorescence, supposée être proportionnelle à la quantité de lipides sur les gouttes, est seulement située à la surface des émulsions, la fluorescence moyenne par objet s'exprime selon l'équation suivante (Eq.1) :

$$\langle I \rangle = \frac{I_{Total}}{R^2}$$

et est proportionnelle à la densité moléculaire de surface $\Gamma$ les lipides par unité de surface.

3. Titrage de l'adsorption de DOPE-CF à la surface des gouttes

Pour mesurer et caractériser l'équilibre d'adsorption des phospholipides fluorescents modèles à l'interface d'émulsion, des gouttes d'huile de soja de 8 $\mu$m (émulsion C) ont été fonctionnalisées dans une solution aqueuse contenant 10 % v / v de DMSO et une concentration de lipides DOPE-CF allant de 0,1 à 20 équivalents de surface, en suivant le protocole résumé dans la partie Matériels et méthodes, section D). La figure 4 représente l'intensité de fluorescence moyenne par goutte, qui est directement liée à la densité de surface en lipides, en fonction de la concentration en lipides. Celle-ci est exprimée en nombre d'équivalents par rapport à la couverture de la surface par une monocouche de lipides. La courbe correspond à une isotherme de Langmuir vérifiant l'équation suivante (Eq.2) :

$$\langle I \rangle = \langle I_0 \rangle \frac{1}{1 + \dfrac{1}{KC}}$$

où $\langle I_0 \rangle$ est la valeur d'intensité moyenne maximale, C la concentration en lipides exprimée en équivalents et K la constante d'affinité apparente. D'après les résultats obtenus, la constante d'affinité entre DOPE-CF et les gouttes est égale à $K_{DOPE-CF} = 0,6 \pm 0,25$ eq$^{-1}$.

4. Titrage de l'adsorption des lipides biotinylés à la surface de gouttes

Les inventeurs ont montré par le passé que les gouttes d'émulsion peuvent être fonctionnalisées avec des protéines adhésives telles que la streptavidine [2] ou les immunoglobulines [4]. Ceci est habituellement réalisé en insérant d'abord des molécules de DSPE-PEG-biotine à l'interface huile / eau sur lesquelles se greffent ensuite les anti-biotine-igGs ou la streptavidine de manière spécifique.

Des gouttes d'huile de soja de 8 µm ont été mises en suspension dans un tampon PB / Tween 20 CMC contenant 10 % v / v de DMSO et une concentration initiale de DSPE-PEG-biotine allant de 0 à 200 équivalents de surface. Après 90 minutes d'incubation à température ambiante, les gouttes sont rincées selon le protocole indiqué dans la partie Matériels et méthodes, section D).

Pour révéler la présence de DSPE-PEG-biotine non fluorescente et construire la courbe de titrage, on ajoute à la suspension une quantité saturante d'IgGs anti-biotine fluorescentes (5 équivalents de surface). Après 30 minutes d'incubation, les gouttelettes sont rincées (Matériels et méthodes, section F) et leur fluorescence est quantifiée par microscopie. La figure 5 montre que la courbe de titrage, représentée par la densité de fluorescence par rapport à la concentration en lipides biotinylés, suit une isotherme de Langmuir avec une constante d'équilibre apparente $K_{DSPE-PEG-biotine} = 0,03 \pm 0,015$ eq$^{-1}$.

5. Distribution de la fluorescence des gouttes biotinylées sur lesquelles sont greffées des protéines

Des gouttes de 8 µm sont biotinylées par incubation avec 100 éq. de DSPE-PEG-biotine et 10 % v/v de DMSO dans la phase continue. Elles sont ensuite incubées avec 5 équivalents de streptavidine ou 10 équivalents d'igGs anti-biotine. Les histogrammes de fluorescence normalisés obtenues sont représentés figures 6A (DSPE-PEG-biotine-streptavidine) et 6B (DSPE-PEG-biotine-igG). Le CV est égal à 14 % et 16 % respectivement.

6. Titrage du greffage de protéines sur les gouttes biotinylées

Connaissant la constante d'association apparente pour la DSPE-PEG-biotine, les courbes de titrage liées au greffage spécifique de la streptavidine et des IgGs anti-biotine aux gouttes biotinylées ont été déterminées. Dans un premier temps, les gouttes d'huile de soja (8 µm, émulsion C) sont biotinylées en utilisant une solution tampon PB / Tween 20 CMC contenant du DMSO à 10 % v/v et contenant 100 équivalents de DSPE-PEG-biotine, concentration 3 fois plus grande que celle correspondant à la constante d'association de ces lipides biotinylés. Les gouttes sont incubées pendant 30 minutes (ce qui est beaucoup plus long que le temps nécessaire pour atteindre l'équilibre au vu des études cinétiques réalisées reportées dans les figures 8A et 8B), dans des solutions de streptavidine et d'IgG avec des concentrations allant respectivement de 0 à 5 et de 0 à 20 équivalents de surface en protéines. Les figures 7A et 7B montrent que la quantité de protéines à la surface sature au-dessus de 1 éq. de streptavidine et 10 éq. d'igGs respectivement. En utilisant une isotherme de Langmuir comme équation d'ajustement, les constantes d'affinité apparentes des protéines aux gouttes biotinylées suivantes sont déterminées : $K_{Streptavidine} = 6,9 \pm 2,3$ eq$^{-1}$ et $K_{igG} = 0,5 \pm 0,18$ eq$^{-1}$.

7. Influence de la nature et de la concentration du tensioactif

Comme détaillé dans la partie Matériels et Méthodes, section D), la fabrication des gouttes fonctionnalisées implique deux tensioactifs, le premier (Pluronic F68) étant utilisé pendant l'étape d'émulsification, tandis que le second (Tween 20) est utilisé pendant l'étape de fonctionnalisation. Avant la fonctionnalisation, le Pluronic F68 est habituellement éliminé pour atteindre une concentration inférieure à sa CMC. Pour étudier l'influence de la présence de tensioactifs au cours de l'étape de biotynilation, l'intensité de fluorescence moyenne a été mesurée pour des gouttes greffées d'IgG, pour lesquelles les types et les concentrations de tensioactifs en présence de DSPE-PEG-biotine lors de l'étape de fonctionnalisation ont été variés. Le tableau 1 ci-dessous montre la fluorescence moyenne et l'écart-type mesurés pour des gouttes recouvertes d'IgGs, qui ont été préalablement fonctionnalisées par la DSPE-PEG-Biotine en l'absence de tensioactif dans la solution de phospholipides (échantillon 1), en présence de Pluronic F68 ou de Tween 20 à des concentrations égales à leur CMC respective (échantillons 2 et 4) ou beaucoup plus élevées que leur CMC (échantillons 3 et 5). Après incubation et rinçage, l'étape de greffage des igGs a été réalisée dans tous les cas avec un tampon PB/Tween 20 CMC.

Tableau 1 : Influence de la nature et de la concentration du tensioactif utilisé lors de l'étape de biotinylation sur la distribution de fluorescence des gouttes greffées d'igGs (diamètre = 5,3 ± 1 $\mu$m). Les concentrations massiques de Pluronic F68 et Tween 20 sont exprimées en fonction de leur concentration micellaire critique (CMC). L'échantillon 3 a été choisi comme référence pour faciliter la comparaison des résultats.

| Echantillon | Tensioactif | Conc. | Fluorescence moyenne (relativement à l'échantillon 3) | Ecart type | CV (%) |
|---|---|---|---|---|---|
| **1** | aucun | - | 13.90 | 2,47 | 17,8 % |
| **2** | Pluronic F68 | CMC | 13.15 | 2,58 | 19,6 % |
| **3** | Pluronic F68 | 15xCMC | 1.00 | 0,09 | 9,1 % |
| **4** | Tween 20 | CMC | 14.81 | 2,48 | 16,8 % |
| **5** | Tween 20 | 10xCMC | 2.86 | 0,56 | 19,5 % |

Les résultats obtenus montrent que la présence d'un tensioactif à haute concentration, qu'il s'agisse du Pluronic F68 ou du Tween 20, est préjudiciable à la fonctionnalisation de l'interface des gouttes par les lipides biotinylés. En revanche, pour de faibles concentrations de tensioactifs, les intensités de fluorescence sont maximisées, ne dépendent pas de la nature du tensioactif utilisé et sont similaires à celles mesurées en l'absence de tensioactif (tampon PB seul).

## 8. Influence de la nature du co-solvant sur l'adsorption des lipides

Pour étudier l'influence de la nature du co-solvant sur l'adsorption des lipides à la surface des gouttes, plusieurs solvants aprotiques polaires usuels ont été utilisés pour préparer les solutions de fonctionnalisation : acétate d'éthyle, pyridine, butanone, DMF, acétonitrile, triéthylamine et DMSO. Toutes les expériences ont été réalisées en diluant DOPE-CF ou DSPE-PEG-biotine dans les co-solvants purs, puis en travaillant avec une fraction volumique de co-solvant de 10 % v/v dans le mélange de fonctionnalisation. A l'exception de la triéthylamine dont la présence conduit rapidement à la déstabilisation de l'émulsion, tous les autres co-solvants n'ont pas d'influence visible sur la stabilité des gouttes au cours de l'incubation à température ambiante. Les concentrations en lipides ont été choisies à 2,5 éq. pour DOPE-CF et 10 éq. pour DSPE-PEG-biotine respectivement. Les IgGs fluorescentes ont été utilisées à 5 éq. pour révéler la présence du lipide biotinylé. Les figures 9A et 9B montrent que pour les deux lipides considérés, l'utilisation d'un co-solvant aprotique polaire permet une augmentation importante de l'adsorption des lipides, et cette augmentation dépend légèrement de la valeur du moment dipolaire du co-solvant.

## 9. Influence de la concentration en DMSO sur l'insertion des lipides

Pour le cas particulier du DMSO utilisé comme co-solvant, l'effet de l'augmentation de sa fraction volumique dans la phase continue sur l'adsorption de lipides (DOPE-CF, 2,5 éq.) à la surface des gouttes (8 $\mu$m) a été évalué. La figure 10 montre que la fluorescence totale des gouttes fonctionnalisées par DOPE-CF, mesurée par cytométrie en flux, augmente avec la fraction volumique de DMSO et sature au-dessus de 10 % v/v de co-solvant dans la phase continue.

## 10. Préparation d'une émulsion magnétique

Une émulsion comprenant des gouttes fonctionnalisées par des IgGs et comportant un noyau magnétique a été préparée, en utilisant une phase dispersée composée d'un ferrofluide de nanoparticules de $Fe_2O_3$ en suspension dans de l'huile minérale.

Puisque le chloroforme est très volatil, les nanoparticules sont redispersées dans une phase hydrophobe non volatile qui sera émulsifiée davantage. Après avoir examiné visuellement plusieurs huiles (végétales, minérales, silicones), différentes concentrations de nanoparticules et d'acide oléique, une formulation optimale d'un ferrofluide stable dans le temps a été déterminée, composée d'huile minérale, de 10 % v/v du ferrofluide initial (1,57 M) et de 5 % v/v d'acide oléique. Les gouttes magnétiques ont ensuite été produites à l'aide d'un dispositif de focalisation de flux microfluidique, contrôlé par pression, ayant des canaux de section transversale rectangulaire égale à $10 \times 12\ \mu\mathrm{m}$, représenté sur la figure 11.

Après émulsification, les gouttes sont fonctionnalisées avec des phospholipides biotinylés et des igGs fluorescentes selon les protocoles décrits dans la partie Matériels et Méthodes, sections D) et E). L'homogénéité de la fluorescence au sein de l'échantillon est très élevée (Fig. 13A), avec un CV proche de 10 % comme le montre la figure 12B. Lors de l'application d'un champ magnétique statique en utilisant un aimant permanent commercial, les gouttes s'alignent (Fig. 13B) grâce à la nature superparamagnétique de leur noyau, ce qui est le comportement attendu pour de tels objets.

### 11. Cytométrie en flux : diagramme FL-SSC

Un diagramme de densité représentant la fluorescence (FL) en fonction de la diffusion latérale (SSC) a été tracé pour des gouttes fonctionnalisées par DSPE-PEG-biotine, sur lesquelles sont ensuite greffées des IgGs fluorescentes selon les protocoles décrits dans la partie Matériels et Méthodes, sections D) et E).

On constate (Fig. 14A) que pour une valeur de SSC donnée, la gamme de fluorescence mesurée est étroite.

## Résultats comparatifs

### 12. Préparation de gouttes fonctionnalisées suivant un protocole de l'art antérieur

A des fins comparatives, des gouttes d'émulsion ont été préparées en utilisant un protocole décrit dans l'art antérieur [4], au cours duquel les lipides sont introduits dans l'huile avant émulsification : Une solution de DSPE-PEG-biotine dans le chloroforme a été préparée, et la quantité nécessaire pour atteindre une concentration en lipides de 0,05 mg.mL$^{-1}$ a été ajoutée dans l'huile de soja. Après évaporation du chloroforme de l'huile par chauffage et sonication, des gouttes monodisperses ont été générées avec l'appareil de Couette, puis des IgGs anti-biotine fluorescentes ont été greffées à leur surface.

### 13. Cytométrie en flux : diagramme FL-SSC

Un diagramme de densité représentant la fluorescence (FL) en fonction de la diffusion latérale (SSC) a été tracé pour les gouttes fonctionnalisées selon le protocole décrit dans l'art antérieur (voir section 12).

On constate (Fig. 14B) que le signal optique fluorescent est beaucoup plus diffus que celui obtenu en fonctionnalisant les gouttes selon le procédé de l'invention.

### 14. Distribution de la fluorescence des gouttes

La comparaison des images obtenues par microscopie à épifluorescence à champ large pour les gouttes fonctionnalisées selon le protocole de l'art antérieur (Fig. 15A) avec celles obtenues pour les gouttes fonctionnalisées selon le procédé de l'invention (Fig. 3C) montre que ce dernier permet d'aboutir à une plus grande homogénéité de fonctionnalisation.

Ceci est confirmé par l'histogramme de fluorescence des gouttes fonctionnalisées selon le protocole de l'art antérieur (Fig. 15B), qui suit une distribution log-normale, avec un facteur d'environ dix entre la goutte la moins fluorescente et la plus fluorescente.

Pour comparaison, la distribution de fluorescence obtenue avec le procédé de l'invention est plus étroite et suit une distribution normale, avec un CV toujours inférieur à 20 % (Fig. 6B).

## Application à l'étude de phagocytose de gouttes d'émulsions halogénées fonctionnalisées

### Étape 1 : Préparation des cellules

Les macrophages murins RAW 264.7 achetés auprès de l'ECACC sont cultivés dans des flasques non traitées T-80 (VWR) remplies de DMEM 4,5 g/L L-glucose supplémenté avec 10 % v/v de Sérum de Veau Foetal et 1 % de Penicilline Streptomycine, dans un incubateur à 37 °C et 5 % $CO_2$. 24h avant l'expérience, elles sont détachées avec du TryPLE et une agitation mécanique, et ensemencées sur dans des boites de Pétri à fond de verre optique (Fluorodish F35-100, WPI) à raison de $10^6$ cellules par lamelle. Une représentation schématique de ces boites de Pétri est présentée sur la figure 16A.

Étape 2 : <u>Préparation des cibles</u>

Des gouttes d'huile végétale halogénée, plus dense que la phase continue aqueuse, ont été utilisées.

*2.1 : Préparation des gouttes d'huile*

Des phospholipides biotinylés DSPE-PEG2000-biotin sont dissous dans de l'huile de soja à 0,05 mg/mL. La phase aqueuse de l'émulsion est constituée de 15 % p/p de F68, et 0.5-2 % p/p d'alginate de sodium selon la taille des gouttes souhaitée. L'huile halogénée est ajoutée goutte à goutte dans la phase aqueuse en agitant doucement à la spatule afin d'obtenir une émulsion grossière, jusqu'à atteindre 75 % d'huile en masse. Cette émulsion est ensuite cisaillée dans un rhéomètre de Couette à 5000 s$^{-1}$, ce qui permet de réduire la distribution de taille des gouttes. L'émulsion cisaillée est diluée afin d'atteindre une fraction massique d'huile de 5 % et de F-68 de 1 %. Cette suspension est décantée plusieurs fois avec une solution de F-68 1% afin d'éliminer les petites gouttes résiduelles.

*2.2 : Fonctionnalisation des cibles*

Il a été choisi d'utiliser 2.10$^6$ cibles/lamelle.
Les cibles sont lavées et centrifugées 3 fois avec un tampon phosphate 0.007 % Tween 20. Elles sont ensuite incubées avec des anticorps anti-biotine Alexa 488, à raison de 1 anticorps pour 1 DSPE-PEG2000-biotin présent à la surface des gouttes pendant 45 minutes à température ambiante. Elles sont ensuite lavées à nouveau 3 fois avec le tampon phosphate 0.007 % Tween20, avant de revenir à une suspension finale de 2.10$^5$ cibles/$\mu$L.

Étape 3 : <u>Mise en contact des cibles et des cellules.</u>

Immédiatement avant la mise en contact, pour chaque lamelle de cellules, 10 $\mu$L de cibles à la concentration de 2.10$^5$ cibles/$\mu$L sont suspendues dans 30 $\mu$L de DMEM 4.5 g/L L-glucose sans rouge de phénol à 37 °C. Les lamelles de cellules ensemencées 24h plus tôt sont rincées avec 1 mL de DMEM 4.5g/L L-glucose sans rouge de phénol à 37 °C.
La suspension de cibles est injectée dans la chambre. Les chambres sont mises à incuber à 37 °C et 5 % CO$_2$ pendant 45 minutes. Dans le cas des gouttes d'huile, la lamelle ensemencées de cellules doit être au-dessus, afin que les gouttes moins denses montent à leur contact, à l'inverse dans le cas des billes de polystyrène la lamelle ensemencée doit être en bas pour que les billes sédimentent à leur contact.
Après 45 minutes, les chambres rincées au PBS de manière à éliminer toutes les gouttes qui ne sont pas internalisées, et fixées avec une solution de paraformaldéhyde 4% pendant 20 minutes à température ambiante.

Étape 4 : <u>Fixation</u>

Les lamelles sont d'abords incubées avec 50 mM de NH$_4$Cl pendant 2 minutes, puis perméabilisées au Triton X-100 0.1 % v/v 10 minutes, et saturées avec une solution d'albumine de sérum bovin 4 % p/p. Elles sont incubées avec 1 $\mu$g/lamelle d'anticorps de rat anti-Cd16/Cd32 de souris pendant 15 minutes, puis rincées à la solution 4 % BSA.

Étape 5 : <u>Observation</u>

Les échantillons fixés sont observés sur un microscope confocal Leica TCS SP8, avec un objectif à immersion à huile 40x, et l'image est prise par 2 détecteurs hybrides Leica. Des lasers à 405, 488, 552 et 638 nm permettent d'éclairer les fluorophores présents, la lumière émise par l'échantillon n'est pas filtrée mais séparée par un prisme. Les fenêtres de longueur d'ondes recueillies sont déterminées au cas par cas en fonction des fluorophores présents afin de minimiser les interférences entre les signaux.
Les figures 16B et 16C montrent deux images représentatives des expériences de phagocytose des gouttes d'émulsions dont le protocole de fonctionnalisation est décrit ci-dessus. Les gouttes sont fonctionnalisées par des lipides biotinylés (Fig. 16B) puis par des anticorps anti-biotin (Fig.16C) reconnus pas des récepteurs des macrophages impliqués dans la phagocytose. Le nombre de gouttes internalisées est plus important dans la Fig. 16C que dans la Fig. 16B qui constitue l'expérience contrôle.

## **REFERENCES**

[1] L.-L. Pontani, I. Jorjadze, V. Viasnoff, J. Brujic, Proc. Natl. Acad. Sci. U. S. A. 109 (2012) 9839.

[2] J. Fattaccioli, J. Baudry, N. Henry, F. Brochard-Wyart, J. Bibette, Soft Matter 4 (2008) 2434.

[3] N. Bourouina, J. Husson, F. Waharte, R.B. Pansu, N. Henry, Soft Matter 7 (2011) 9130.

[4] K. Ben M'Barek, D. Molino, S. Quignard, M. Plamont, Y. Chen, P. Chavrier, J. Fattaccioli, Biomaterials 51 (2015) 270.

[5] M. Hadorn, E. Boenzli, K.T. Sorensen, H. Fellermann, P. Eggenberger Hotz, M.M. Hanczyc, P. Eggenberger, M.M. Hanczyc, P. Eggenberger Hotz, Proc. Natl. Acad. Sci. U. S. A. 109 (2012) 20320.

[6] L. Feng, L.-L. Pontani, R. Dreyfus, P. Chaikin, J. Brujic, Soft Matter 9 (2013) 9816.

[7] A.R. Thiam, N. Bremond, J. Bibette, Langmuir 28 (2012) 6291.

[8] O. Campés, T. Mammoto, S. Hasso, R. a Sperling, D. O'Connell, A.G. Bischof, R. Maas, D. a Weitz, L. Mahadevan, D.E. Ingber, Nat. Methods 11 (2013) 183.

[9] T. Mason, J. Bibette, Phys. Rev. Lett. 77 (1996) 3481.

[10] K.L. Mittal, J. Pharm. Sci. 61 (1972) 1334.

[11] N.V. Efremova, B. Bondurant, D.F. O'Brien, D. Leckband, Biochemistry 39 (2000) 3441.

[12] D.S. Banks, C. Fradin, Biophys. J. 89 (2005) 2960.

[13] T.C. Werner, J.R. Bunting, R.E. Cathou, Proc. Natl. Acad. Sci. U. S. A. 69 (1972) 795.

[14] Y. Xia, G.M. Whitesides, Angew. Chemie Int. Ed. 37 (1998) 550.

[15] R. Massart, IEEE Trans. Magn. 17 (1981) 1247.

## Revendications

1. Colloïde comprenant des particules colloïdales liquides fonctionnalisées en suspension dans un liquide, **caractérisé en ce que** :

   - le liquide est constitué d'une solution aqueuse comprenant un solvant aprotique polaire, la fraction volumique dudit solvant aprotique polaire dans ledit liquide étant comprise entre 1 et 15 %, de préférence entre 5 et 15 %, avantageusement entre 8 et 15 % ; et
   - les particules colloïdales liquides fonctionnalisées consiste en des gouttes d'huile à la surface desquelles sont adsorbés des lipides.

2. Colloïde comprenant des particules colloïdales liquides fonctionnalisées selon la revendication 1, **caractérisé en ce que**, au sein d'une sous-population de particules ayant un diamètre égal à d +/- 15 %, le coefficient de variation de la quantité de lipide adsorbés à la surface des gouttes est inférieur ou égal à 20 %.

3. Colloïde comprenant des particules colloïdales liquides fonctionnalisées selon la revendication 2, **caractérisé en ce que** le coefficient de variation est déterminé par microscopie en épifluorescence à champ large.

4. Colloïde comprenant des particules colloïdales liquides fonctionnalisées selon l'une quelconque des revendications 1 à 3, **caractérisé en ce que** le solvant aprotique polaire est sélectionné dans le groupe constitué du DMSO, de l'acétate d'éthyle, de l'acétonitrile, de la pyridine, de la butanone, de la triéthylamine, du DMF, et de leurs mélanges, de préférence il s'agit du DMSO.

5. Colloïde comprenant des particules colloïdales liquides fonctionnalisées selon l'une quelconque des revendications 1 à 4, **caractérisé en ce que** l'huile est une huile minérale, une huile végétale telle que l'huile de soja, une huile siliconée, une huile halogénée, ou un mélange de celles-ci.

6. Colloïde comprenant des particules colloïdales liquides fonctionnalisées selon l'une quelconque des revendications 1 à 5, **caractérisé en ce que** les gouttes d'huile sont des gouttes d'huile minérale et comprennent des nanoparticules de $Fe_2O_3$ en suspension.

7. Colloïde comprenant des particules colloïdales liquides fonctionnalisées selon l'une quelconque des revendications 1 à 6, **caractérisé en ce que** les lipides comportent au moins un groupement fluorophore et/ou au moins une biomolécule, telle qu'une biotine, optionnellement greffé(e) au moyen d'un linker tel qu'une chaîne PEG.

8. Colloïde comprenant des particules colloïdales liquides fonctionnalisées selon l'une quelconque des revendications 1 à 7, **caractérisé en ce que** les lipides sont des phospholipides, en particulier des phosphatidyléthanolamines telles que DOPE ou DSPE, éventuellement greffés d'au moins un groupement fluorophore et/ou d'au moins une biomolécule, optionnellement au moyen d'un linker.

9. Colloïde comprenant des particules colloïdales liquides fonctionnalisées selon la revendication 7 ou 8, **caractérisé en ce que** la biomolécule est une vitamine, telle que la biotine, un peptide, tel que l'acide arginyl glycyl aspartique (RGD), une protéine, telle que la streptavidine, une enzyme, un anticorps, notamment une immunoglobuline de type G (igG) ou une association de celles-ci, la biomolécule étant optionnellement fluorescente.

10. Colloïde comprenant des particules colloïdales liquides fonctionnalisées selon l'une quelconque des revendications 1 à 9, **caractérisé en ce que** les lipides sont sélectionnés dans le groupe constitué de DOPE-CF, DSPE-PEG-Biotine et leurs mélanges.

11. Utilisation d'un colloïde comprenant des particules colloïdales liquides fonctionnalisées selon l'une quelconque des revendications 1 à 10 pour le calibrage d'un cytomètre en flux.

12. Utilisation d'un colloïde comprenant des particules colloïdales liquides fonctionnalisées selon l'une quelconque des revendications 1 à 10 pour l'étude de leur ingestion par des cellules, telle que la phagocytose par des macrophages, **caractérisée en ce que** la densité de l'huile constitutive des particules colloïdales liquides fonctionnalisées est supérieure à 1.

**Fig. 1**

**Fig. 2A**

Fig. 2B

Fig. 2C

Fig. 3A

Fig. 3B

Fig. 3C

Fig. 3D

Fig. 3E

Fig. 4

Fig. 5

**Fig. 6A**

**Fig. 6B**

Fig. 7A

Fig. 7B

Fig. 8A

Fig. 8B

Fig. 9A

1 - Acétate d'éthyle
2 - Pyridine
3 - Butanone
4 - DMF
5 - Acetonitrile
6 - DMSO

Fig. 9B

**Fig. 10A**

**Fig. 10B**

**Fig. 11**

**Fig. 12A**

**Fig. 12B**

Fig. 13A

Fig. 13B

Fig. 14A

Fig. 14B

**Fig. 15A**

**Fig. 15B**

Fig. 16A

Fig. 16B

Fig. 16C

## RÉFÉRENCES CITÉES DANS LA DESCRIPTION

### Littérature non-brevet citée dans la description

- **OBEY ; VINCENT.** *J. Colloid Interface Sci.,* 1994, vol. 163, 454
- *CHEMICAL ABSTRACTS,* 8001-22-7
- *CHEMICAL ABSTRACTS,* 8042-47-5
- *CHEMICAL ABSTRACTS,* 9003-11-6
- *CHEMICAL ABSTRACTS,* 9005-64-5
- *CHEMICAL ABSTRACTS,* 112-80-1
- *CHEMICAL ABSTRACTS,* 9005-38-3
- **L.-L. PONTANI ; I. JORJADZE ; V. VIASNOFF ; J. BRUJIC.** *Proc. Natl. Acad. Sci. U. S. A.,* 2012, vol. 109, 9839
- **J. FATTACCIOLI ; J. BAUDRY ; N. HENRY ; F. BROCHARD-WYART ; J. BIBETTE.** *Soft Matter,* 2008, vol. 4, 2434
- **N. BOUROUINA ; J. HUSSON ; F. WAHARTE ; R.B. PANSU ; N. HENRY.** *Soft Matter,* 2011, vol. 7, 9130
- **K. BEN M'BAREK ; D. MOLINO ; S. QUIGNARD ; M. PLAMONT ; Y. CHEN ; P. CHAVRIER ; J. FATTACCIOLI.** *Biomaterials,* 2015, vol. 51, 270
- **M. HADORN ; E. BOENZLI ; K.T. SORENSEN ; H. FELLERMANN ; P. EGGENBERGER HOTZ ; M.M. HANCZYC ; P. EGGENBERGER ; M.M. HANCZYC ; P. EGGENBERGER HOTZ.** *Proc. Natl. Acad. Sci. U. S. A.,* 2012, vol. 109, 20320
- **L. FENG ; L.-L. PONTANI ; R. DREYFUS ; P. CHAIKIN ; J. BRUJIC.** *Soft Matter,* 2013, vol. 9, 9816
- **A.R. THIAM ; N. BREMOND ; J. BIBETTE.** *Langmuir,* 2012, vol. 28, 6291
- **O. CAMPÉS ; T. MAMMOTO ; S. HASSO ; R. A SPERLING ; D. O'CONNELL ; A.G. BISCHOF ; R. MAAS ; D. A WEITZ ; L. MAHADEVAN ; D.E. INGBER.** *Nat. Methods,* 2013, vol. 11, 183
- **T. MASON ; J. BIBETTE.** *Phys. Rev. Lett.,* 1996, vol. 77, 3481
- **K.L. MITTAL.** *J. Pharm. Sci,* 1972, vol. 61, 1334
- **N.V. EFREMOVA ; B. BONDURANT ; D.F. O'BRIEN ; D. LECKBAND.** *Biochemistry,* 2000, vol. 39, 3441
- **D.S. BANKS ; C. FRADIN.** *Biophys. J.,* 2005, vol. 89, 2960
- **T.C. WERNER ; J.R. BUNTING ; R.E. CATHOU.** *Proc. Natl. Acad. Sci. U. S. A.,* 1972, vol. 69, 795
- **Y. XIA ; G.M. WHITESIDES.** *Angew. Chemie Int. Ed.,* 1998, vol. 37, 550
- **R. MASSART.** *IEEE Trans. Magn.,* 1981, vol. 17, 1247